# EUROPEAN PATENT APPLICATION

(11) **EP 4 477 298 A1**
(43) Date of publication of application: **18.12.2024**
(21) Application number: 22926106.0
(22) Date of filing: 22.12.2022
(51) Int. Cl.: B01D 63/00, B01D 63/06, C07C 29/152, C07C 31/04, B01D 53/22

(54) **SEPARATION MEMBRANE MODULE**

(30) Priority: 08.02.2022 JP 2022017955; 25.08.2022 JP 2022134438
(71) Applicant: NGK Insulators, Ltd., Nagoya-shi, Aichi 467-8530 (JP)
(72) Inventor: NAKAGAWA, Kosuke, Nagoya-shi, Aichi 467-8530 (JP); IIDA, Kazuki, Nagoya-shi, Aichi 467-8530 (JP); KAN, Hirofumi, Nagoya-shi, Aichi 467-8530 (JP); TORII, Atsushi, Nagoya-shi, Aichi 467-8530 (JP)
(74) Representative: Müller-Boré & Partner Patentanwälte PartG mbB
(86) International application number: PCT/JP2022/047357
(87) International publication number: WO 2023/153092

(57) **Abstract**

A separation membrane module (1) includes: a monolith type reactor (1); a housing (3); an annular first sealing portion (4) sealing a gap between the housing (3) and a first end portion (1a) of the reactor; and an annular second sealing portion (5) sealing a gap between the housing (3) and a second end portion (1b) of the reactor. The first sealing portion (4) is deformable or movable in a longitudinal direction together with the reactor (1). The second sealing portion (5) fixes the reactor (1) to the housing (5).

## Description

### TECHNICAL FIELD

The present invention relates to a separation membrane module.

### BACKGROUND ART

Recent years have seen development of reactors that can increase the conversion efficiency in a conversion reaction for converting a source gas containing hydrogen and carbon oxide into a liquid fuel such as methanol or ethanol (specifically, a fuel that is in a liquid state at normal temperature and normal pressure) by separating water vapor generated together with the liquid fuel.

For example, Patent Literature 1 disclosures a tube type reactor including: a separation membrane that allows water vapor, which is one of products of the conversion reaction, to pass therethrough; a non-permeation-side flow path through which the source gas flows; and a catalyst filling the flow path.

### CITATION LIST

### Patent Literature

Patent Literature 1: JP 2018-8940A

### SUMMARY

### TECHNICAL PROBLEM

Incidentally, it is conceivable to increase the area of the separation membrane per unit volume by using a monolith type reactor that includes a plurality of flow paths extending through the reactor in a longitudinal direction.

However, when the reactor is fixed in a housing and caused to operate, there is a risk of the separation membrane module being damaged due to a difference between the coefficient of thermal expansion of the reactor and the coefficient of thermal expansion of the housing. Specifically, there is a problem that the reactor itself or a sealing portion that seals a gap between the reactor and the housing is damaged. Such a problem also occurs in a case where the separation membrane module includes a separation filter instead of the reactor.

An object of the present invention is to provide a separation membrane module that can be kept from being damaged.

### SOLUTION TO PROBLEM

A separation membrane module according to the present invention includes: a monolith type reactor extending in a longitudinal direction and configured to be used for a conversion reaction for converting a source gas into a liquid fuel, the source gas containing hydrogen and carbon oxide; a housing in which the reactor is housed; an annular first sealing portion sealing a gap between the housing and a first end portion of the reactor in the longitudinal direction; and an annular second sealing portion sealing a gap between the housing and a second end portion of the reactor in the longitudinal direction. The first sealing portion is deformable or movable in the longitudinal direction together with the reactor. The second sealing portion fixes the reactor to the housing.

### ADVANTAGEOUS EFFECTS

According to the present invention, it is possible to provide a separation membrane module that can be kept from being damaged.

### BRIEF DESCRIPTION OF DRAWINGS

FIG. 1 is a perspective view of a reactor 1 according to an embodiment.
FIG. 2 is a cross-sectional view taken along the line A-A shown in FIG. 1.
FIG. 3 is a cross-sectional view taken along the line B-B shown in FIG. 1.
FIG. 4 is a cross-sectional view taken along the line C-C shown in FIG. 2.
FIG. 5 is a transparent side view of a separation membrane module according to a first embodiment.
FIG. 6 is a transparent side view of a separation membrane module according to a second embodiment.
FIG. 7 is a transparent side view of a separation membrane module according to a third embodiment.
FIG. 8 is a cross-sectional view of a separation membrane module according to Variation 3.
FIG. 9 is a cross-sectional view of the separation membrane module according to Variation 3.
FIG. 10 is a schematic view of a separation membrane module according to Variation 4.
FIG. 11 is a schematic view of the separation membrane module according to Variation 3.

### DESCRIPTION OF EMBODIMENTS

### 1. First Embodiment

The following describes a first embodiment of the present invention with reference to the drawings. Note that the drawings are schematic drawings, and dimensional ratios and the like may differ from actual ratios and the like.

### Reactor 1

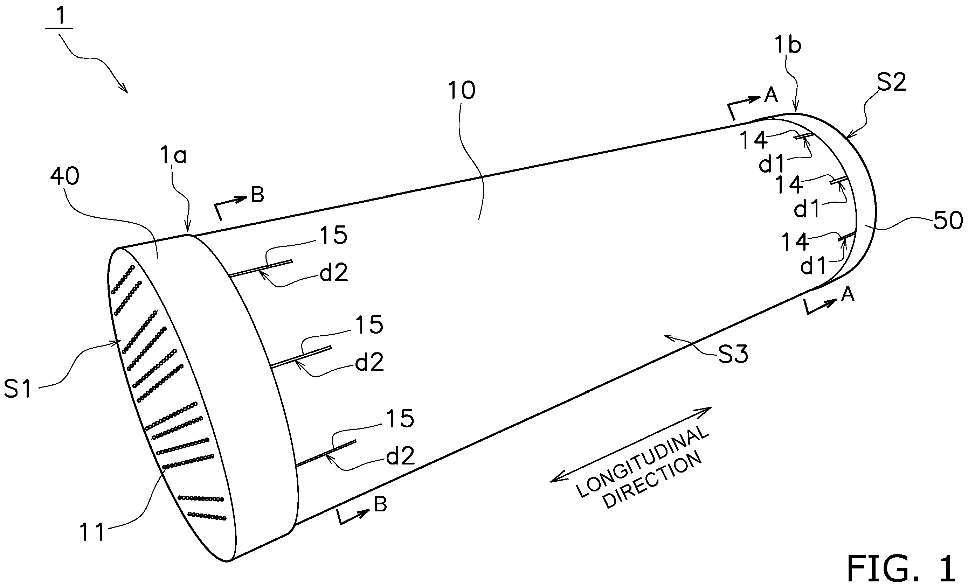

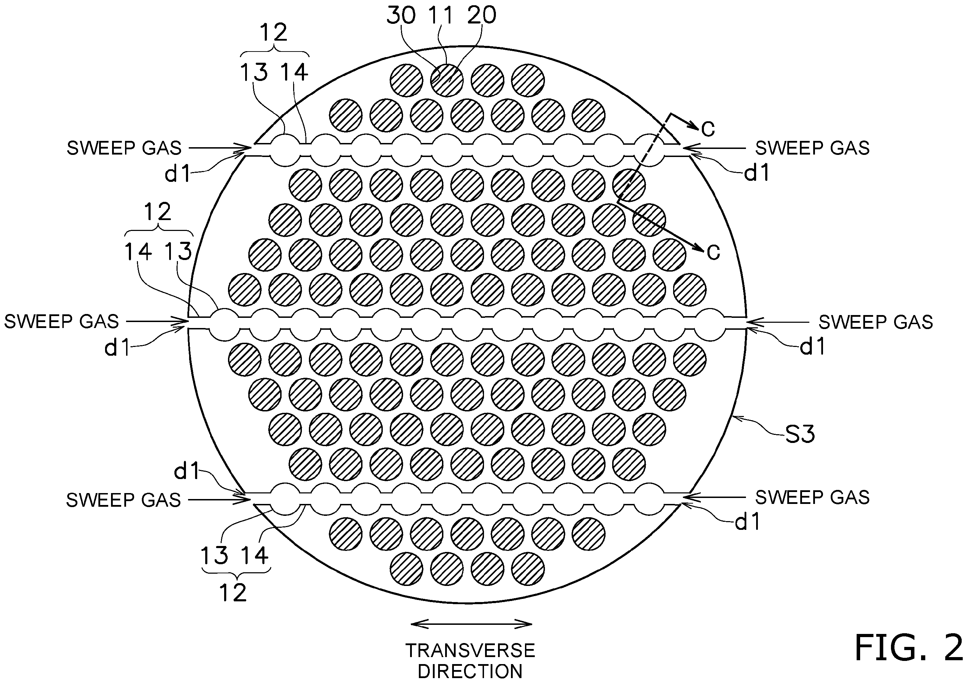

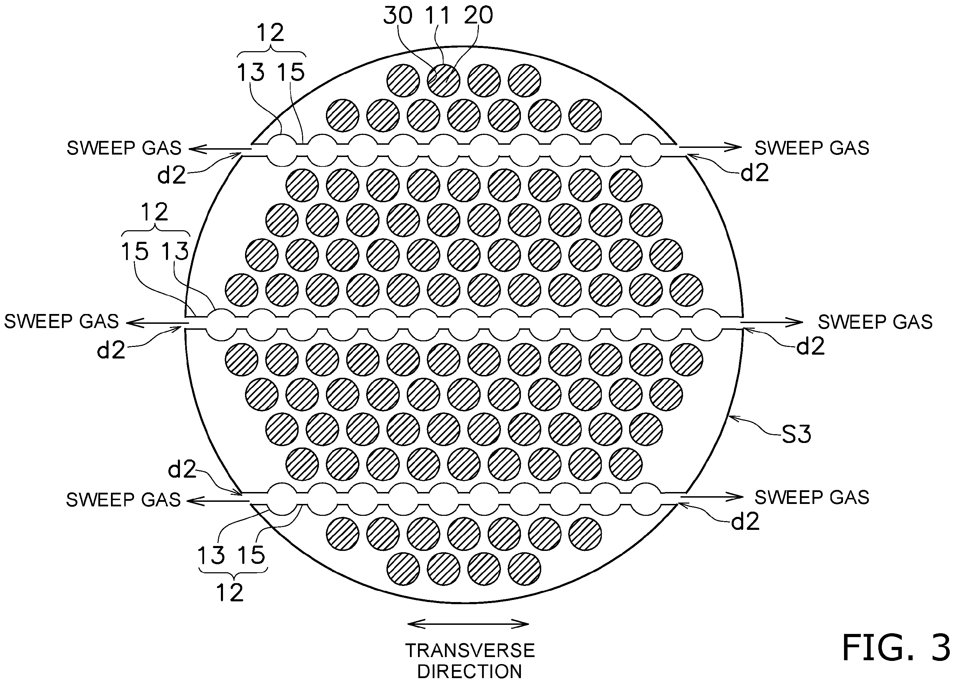

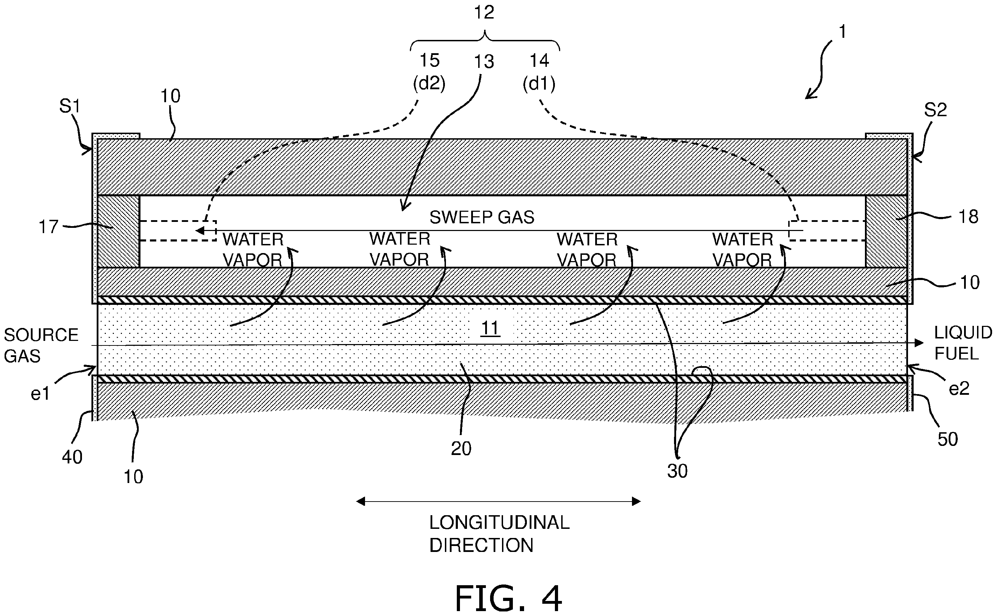

FIG. 1 is a perspective view of a reactor 1. FIG. 2 is a cross-sectional view taken along the line A-A shown in FIG. 1. FIG. 3 is a cross-sectional view taken along the line B-B shown in FIG. 1. FIG. 4 is a cross-sectional view taken along the line C-C shown in FIG. 2.

The reactor 1 is a so-called membrane reactor for converting a source gas into a liquid fuel. The source gas contains at least hydrogen and carbon oxide. At least one of carbon monoxide and carbon dioxide can be used as the carbon oxide. The source gas may be a so-called synthesis gas (syngas). The liquid fuel is a fuel that is in a liquid state at normal temperature and normal pressure or a fuel that can be liquefied at normal temperature in a pressurized state. Examples of fuels that are in a liquid state at normal temperature and normal pressure include methanol, ethanol, a liquid fuel represented by CₙH₂₍ₘ₋₂ₙ₎ (m is an integer smaller than 90, and n is an integer smaller than 30), and a mixture of these. Examples of fuels that can be liquefied at normal temperature in a pressurized state include propane, butane, and a mixture of these.

For example, a reaction formula (1) for the synthesis of methanol through catalytic hydrogenation of a source gas containing carbon dioxide and hydrogen in the presence of a catalyst is expressed as follows.

CO₂+3H₂ ⇔ CH₃OH+H₂O (1)

The above reaction is an equilibrium reaction, and in order to increase both the conversion efficiency and the reaction rate, the reaction is preferably carried out at a high temperature and a high pressure (e.g., 180°C or higher and 2 MPa or higher). The liquid fuel is in a gaseous state when it is synthesized, and remains in the gaseous state at least until the liquid fuel flows out from the reactor 1. It is preferable that the reactor 1 has heat resistance and pressure resistance suitable for synthesis conditions of the desired liquid fuel.

As shown in FIG. 1, the reactor 1 has a monolith shape. The term "monolith" means a shape that includes a plurality of holes extending through the reactor in the longitudinal direction, and encompasses a honeycomb shape. The reactor 1 extends in the longitudinal direction. The reactor 1 has a columnar shape. In the present embodiment, the reactor 1 has a circular column shape, but the external shape of the reactor 1 is not particularly limited.

The reactor 1 includes a first end portion 1a and a second end portion 1b. The first end portion 1a extends from an end of the reactor 1 in the longitudinal direction by a length of 2/5 of the entire length of the reactor 1 in the longitudinal direction. The second end portion 1b extends from the other end of the reactor 1 in the longitudinal direction by a length of 2/5 of the entire length of the reactor 1 in the longitudinal direction. In the present embodiment, the source gas flows into the reactor 1 from the first end portion 1a side, and the liquid fuel flows out of the reactor 1 from the second end portion 1b side.

The reactor 1 has a first end surface S1, a second end surface S2, and a side surface S3. The first end surface S1 is the end surface on the first end portion 1a side. The second end surface S2 is the end surface on the second end portion 1b side. The first end surface S1 is opposite to the second end surface S2. The side surface S3 is continuous to outer edges of the first end surface S1 and the second end surface S2.

As shown in FIGS. 1 to 4, the reactor 1 includes a porous support 10, a catalyst 20, a separation membrane 30, a first seal portion 40, and a second seal portion 50.

The porous support 10 is a columnar body extending in the longitudinal direction of the reactor 1. The porous support 10 is constituted by a porous material.

A ceramic material, a metallic material, a resin material, or the like can be used as the porous material, and a ceramic material is particularly favorable. At least one of alumina (Al₂O₃), titania (TiO₂), mullite (Al₂O₃·SiO₂), potsherd, and cordierite (Mg₂Al₄Si₅O₁₈) can be used as an aggregate for the ceramic material. At least one of titania, mullite, easily sinterable alumina, silica, glass frit, a clay mineral, and easily sinterable cordierite can be used as an inorganic binder for the ceramic material. However, the ceramic material need not necessarily contain an inorganic binder.

As shown in FIGS. 2 and 3, the porous support 10 includes a large number of first flow paths 11 and a plurality of second flow paths 12.

Each first flow path 11 is formed along the longitudinal direction of the reactor 1 as shown in FIG. 4. Each first flow path 11 is on a non-permeation side of the separation membrane 30. The source gas is caused to flow through the first flow paths 11. Each first flow path 11 is a through hole. Each first flow path 11 is open in the first end surface S1 and the second end surface S2 of the reactor 1. Each first flow path 11 includes an inlet port e1 for the source gas formed in the first end surface S1 and an outlet port e2 for the liquid fuel formed in the second end surface S2.

The catalyst 20 is disposed inside each first flow path 11. The number of first flow paths 11, their positions, shape, and the like can be changed as appropriate.

Each second flow path 12 is on a permeation side of the separation membrane 30. A sweep gas for sweeping water vapor that has passed through the separation membrane 30 is caused to flow through the second flow paths 12. Inert gas (e.g., nitrogen), air, or the like can be used as the sweep gas. The number of second flow paths 12, their positions, shape, and the like can be changed as appropriate.

As shown in FIGS. 2 and 3, each second flow path 12 is constituted by a plurality of cells 13, an inlet slit 14, and an outlet slit 15.

The plurality of cells 13 are lined up along a line extending in a transverse direction (a direction perpendicular to the longitudinal direction) of the reactor 1. Each cell 13 is formed along the longitudinal direction of the reactor 1 as shown in FIG. 4. Two ends of each cell 13 are respectively sealed by first and second opening sealing portions 17 and 18. The first and second opening sealing portions 17 and 18 can be constituted by the porous material described above.

As shown in FIG. 1, the inlet slit 14 is formed in the second end portion 1b of the reactor 1 in the longitudinal direction. As shown in FIG. 2, the inlet slit 14 is formed along the transverse direction of the reactor 1. The inlet slit 14 extends through the plurality of cells 13. Both ends of the inlet slit 14 are open in the side surface S3. The inlet slit 14 includes a pair of inlet ports d1 formed in the side surface S3. The pair of inlet ports d1 correspond to an end of the second flow path 12 in the longitudinal direction.

As shown in FIG. 1, the outlet slit 15 is formed in the first end portion 1a of the reactor 1 in the longitudinal direction. As shown in FIG. 3, the outlet slit 15 is formed along the transverse direction of the reactor 1. The outlet slit 15 extends through the plurality of cells 13. Both ends of the outlet slit 15 are open in the side surface S3. The outlet slit 15 includes a pair of discharge ports d2 formed in the side surface S3. The pair of discharge ports d2 correspond to the other end of the second flow path 12 in the longitudinal direction.

The catalyst 20 is disposed inside each first flow path 11. The catalyst 20 preferably fills each first flow path 11, but may also be disposed so as to form a layer on the surface of the separation membrane 30. The catalyst 20 promotes the conversion reaction for converting the source gas into the liquid fuel as shown in the above formula (1).

A known catalyst suitable for the conversion reaction for obtaining the desired liquid fuel can be used as the catalyst 20. Examples of the catalyst 20 include metal catalysts (copper, palladium, etc.), oxide catalysts (zinc oxide, zirconia, gallium oxide, etc.), and composites of these (copper-zinc oxide, copper-zinc oxide-alumina, copper-zinc oxide-chromium oxide-alumina, copper-cobalt-titania, and these catalysts modified with palladium, etc.).

The separation membrane 30 is supported by the porous support 10. The separation membrane 30 surrounds the first flow paths 11. The separation membrane 30 is disposed between the first flow paths 11 and the second flow paths 12.

The separation membrane 30 allows water vapor, which is one of the products generated as a result of the conversion reaction for converting the source gas into the liquid fuel, to pass therethrough. Thus, the reaction equilibrium of the above formula (1) can be shifted to the product side by utilizing an equilibrium shift effect.

It is preferable that the separation membrane 30 has a water vapor permeability coefficient of 100 nmol/(s·Pa·m²) or more. The water vapor permeability coefficient can be obtained using a known method (see Ind. Eng. Chem. Res., 40, 163-175(2001)).

It is preferable that the separation membrane 30 has a separation coefficient of 100 or more. The greater the separation coefficient, the easier it is for water vapor to pass through the separation membrane 30 and the more difficult it is for components (hydrogen, carbon dioxide, the liquid fuel, etc.) other than water vapor to pass through the separation membrane 30. The separation coefficient can be obtained using a known method (see FIG. 1 in "Separation and Purification Technology 239 (2020) 116533").

An inorganic membrane can be used as the separation membrane 30. Inorganic membranes have heat resistance, pressure resistance, and water vapor resistance and thus are preferable. Examples of inorganic membranes include zeolite membranes, silica membranes, alumina membranes, and composite membranes thereof. In particular, LTA-type zeolite membranes in which a molar ratio (Si/Al) between silicon element (Si) and aluminum element (Al) is 1.0 or more and 3.0 or less have excellent water vapor permeability and thus are favorable.

As shown in FIG. 1, the first seal portion 40 covers the first end surface S1 and a portion of the side surface S1 of the porous support 10. The first seal portion 40 suppresses intrusion of the source gas into the porous support 10. As shown in FIG. 4, the first seal portion 40 is formed so as not to close the inlet ports e1 of the first flow paths 11. The first seal portion 40 covers the first opening sealing portion 17. The first seal portion 40 can be constituted by glass, metal, rubber, resin, or the like.

As shown in FIG. 1, the second seal portion 50 covers the second end surface S2 and a portion of the side surface S1 of the porous support 10. The second seal portion 50 suppresses intrusion of the liquid fuel into the porous support 10. As shown in FIG. 4, the second seal portion 50 is formed so as not to close the outlet ports e2 of the first flow paths 11. The second seal portion 50 covers the second opening sealing portion 18. The second seal portion 50 can be constituted by glass, metal, rubber, resin, or the like.

### Method for Synthesizing Liquid Fuel Using Reactor 1

The following describes a method for synthesizing the liquid fuel using the reactor 1 with reference to FIG. 4.

The method for synthesizing the liquid fuel using the reactor 1 includes a step of causing the sweep gas to flow through the second flow paths 12 provided on the permeation side of the separation membrane 30 while causing the source gas to flow through the first flow paths 11 provided on the non-permeation side of the separation membrane 30.

The source gas flows into the first flow paths 11 from the inlet ports e1 of the first flow paths 11. In the first flow paths 11, water vapor is generated together with the liquid fuel as shown in the above formula (1). The synthesized liquid fuel flows out from the outlet ports e2 of the first flow paths 11. Water vapor, which is one of the products, passes through the separation membrane 30 and the porous support 10 in this order, and enters the second flow paths 12. However, a remaining portion of the source gas that was not used for the conversion reaction, the produced water vapor, and the like may be mixed in the liquid fuel flowing out from the outlet ports e2.

The sweep gas flows into the inlet ports d1 of the inlet slits 14 and then flows into the cells 13 from the inlet slits 14. Then, the sweep gas that has flowed into the cells 13 from the inlet slits 14 takes on water vapor that has passed through the separation membrane 30, and flows through the cells 13 toward the outlet slits 15 while absorbing reaction heat generated in the conversion reaction. The sweep gas that has reached the outlet slits 15 is discharged from the discharge ports d2 of the outlet slits 15.

As shown in FIG. 4, in the present embodiment, the direction of flows of the sweep gas in the second flow paths 12 is opposite to the direction of flows of the source gas in the first flow paths 11 in a side view of the separation membrane 30. That is, the sweep gas flows through the second flow paths 12 in a direction opposite to the direction in which the source gas flows through the first flow paths 11.

However, the direction of flows of the sweep gas in the second flow paths 12 may be the same as the direction of flows of the source gas in the first flow paths 11 in a side view of the separation membrane 30. That is, the sweep gas may flow through the second flow paths 12 in parallel to the source gas flowing through the first flow paths 11.

### Separation Membrane Module 2

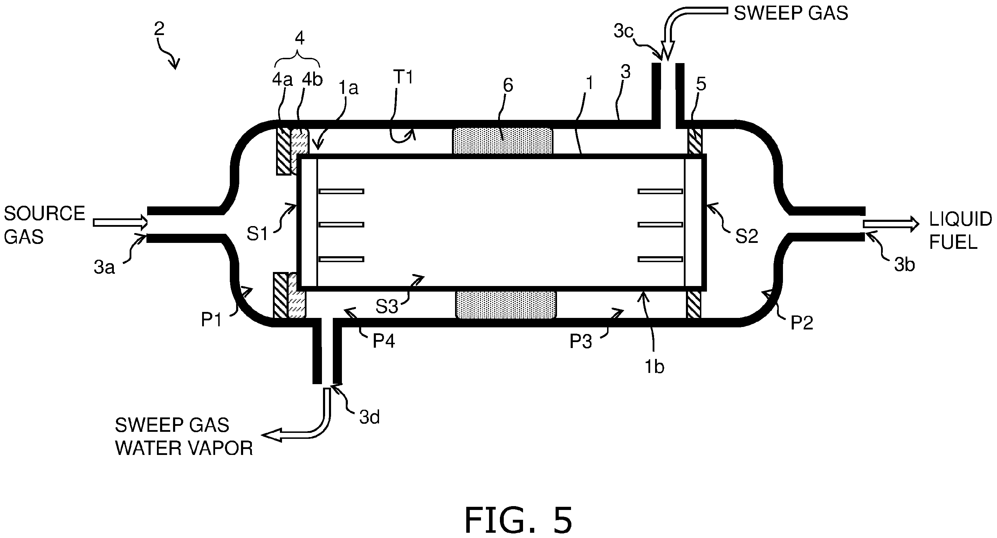

The following describes the separation membrane module 2 according to a first embodiment. FIG. 5 is a transparent side view of the separation membrane module 2.

As shown in FIG. 5, the separation membrane module 2 includes the monolith type reactor 1 described above, a housing 3, an annular first sealing portion 4, an annular second sealing portion 5, and an annular flow stopper 6.

The housing 3 is constituted by a metallic material (e.g., stainless steel), for example. The reactor 1 is housed in the housing 3. The inside of the housing 3 is sectioned into first through fourth spaces P1 to P4 by the first sealing portion 4, the second sealing portion 5, and the flow stopper 6. The housing 3 includes a source gas supply port 3a, a liquid fuel discharge port 3b, a sweep gas supply port 3c, and a sweep gas discharge port 3d.

The source gas is supplied to the first space P1 via the source gas supply port 3a. The source gas flows from the first space P1 into each first flow path 11 (see FIG. 4) of the reactor 1. The liquid fuel flows out from each first flow path 11 of the reactor 1 into the second space P2. The liquid fuel is discharged from the liquid fuel discharge port 3b via the second space P2.

The sweep gas is supplied to the third space P3 via the sweep gas supply port 3c. The sweep gas flows from the third space P3 into each second flow path 12 (see FIG. 4) of the reactor 1. The sweep gas that has taken on water vapor in each second flow path 12 flows from each second flow path 12 of the reactor 1 into the fourth space P4. The sweep gas is discharged from the sweep gas discharge port 3d via the fourth space P4.

In FIG. 5, the sweep gas supply port 3c is provided on the side opposite to the sweep gas discharge port 3d with respect to the axis of the reactor 1. With this configuration, the lengths of paths of the sweep gas flowing from the sweep gas supply port 3c to the sweep gas discharge port 3d via the respective second flow paths 12, which will be described later, can be made equivalent to each other, and accordingly, it is possible to suppress maldistribution of flows of the sweep gas. However, the positional relationship between the sweep gas supply port 3c and the sweep gas discharge port 3d can be changed as appropriate.

The first sealing portion 4 seals a gap between the housing 3 and the first end portion 1a of the reactor 1. The first sealing portion 4 holds the first end portion 1a of the reactor 1. The first sealing portion 4 includes a fixed portion 4a and an elastic portion 4b. The fixed portion 4a is an example of a "first fixed portion" according to the present invention, and the elastic portion 4b is an example of an "elastic portion" according to the present invention.

The fixed portion 4a has an annular shape. The fixed portion 4a is fixed to an inner surface T1 of the housing 3. The fixed portion 4a is disposed in such a manner as to protrude from the inner surface T1 of the housing 3. It is desirable that the fixed portion 4a is made of a material similar to the material of the housing 3, but there is no limitation to this configuration as long as the fixed portion 4a serves as a fixed portion.

The elastic portion 4b has an annular shape. The elastic portion 4b abuts against the fixed portion 4a and the first end surface S1 of the reactor 1. The elastic portion 4b is sandwiched between the fixed portion 4a and the first end surface S1 of the reactor 1.

The elastic portion 4b has elasticity and is deformable in the longitudinal direction. Therefore, when the housing 3 expands or contracts in the longitudinal direction relative to the reactor 1 due to a difference between the coefficient of thermal expansion (CTE) of the reactor 1 and the coefficient of thermal expansion of the housing 3 when the separation membrane module 2 starts to operate or stops operation, the elastic portion 4b deforms (expands or contracts) in response to the expansion or contraction of the housing 3. Since the separation membrane module can cope with the expansion and contraction of the housing 3 while maintaining the holding ability and the sealing ability of the first sealing portion 4, the reactor 1 and the first sealing portion 4 can be kept from being damaged as a result of stress being applied thereto.

The second sealing portion 5 has an annular shape. The second sealing portion 5 fixes the reactor 1 to the housing 3. In the present specification, the description "the second sealing portion 5 fixes the reactor 1 to the housing 3" means that the reactor 1 is positioned in the longitudinal direction by the second sealing portion 5. In the present embodiment, the second end portion 1b of the reactor 1 is positioned in the longitudinal direction by the second sealing portion 5. The second sealing portion 5 is connected to the side surface S3 of the reactor 1 and the inner surface T1 of the housing 3.

The second space P2 side surface of the second sealing portion 5 is exposed to the liquid fuel having a high temperature and water vapor, and therefore, the material of the second sealing portion 5 needs to be resistant to a chemical load applied by the liquid fuel having a high temperature and be resistant to water vapor. Examples of the material of the second sealing portion 5 include glass, silver solder, solder, and inorganic adhesives. Alternatively, the second sealing portion 5 may be constituted by rubber or an elastomer (e.g., fluorocarbon rubber, EPDM rubber, etc.) that has chemical resistance, heat resistance, and water vapor resistance. However, rubbers and plastics that do not have chemical resistance, heat resistance, and water vapor resistance are not suitable for the material of the second sealing portion 5.

The flow stopper 6 has an annular shape. The flow stopper 6 is disposed between the reactor 1 and the housing 3. The flow stopper 6 is disposed between the third space P3 and the fourth space P4. The flow stopper 6 suppresses flows of the sweep gas from the third space P3 to the fourth space P4. However, it is sufficient that the flow stopper 6 is capable of suppressing flows of the sweep gas, and need not serve as a hermetic seal between the reactor 1 and the housing 3. The flow stopper 6 can be constituted by expanded graphite, rubber, resin, or the like, for example.

### 3. Second Embodiment

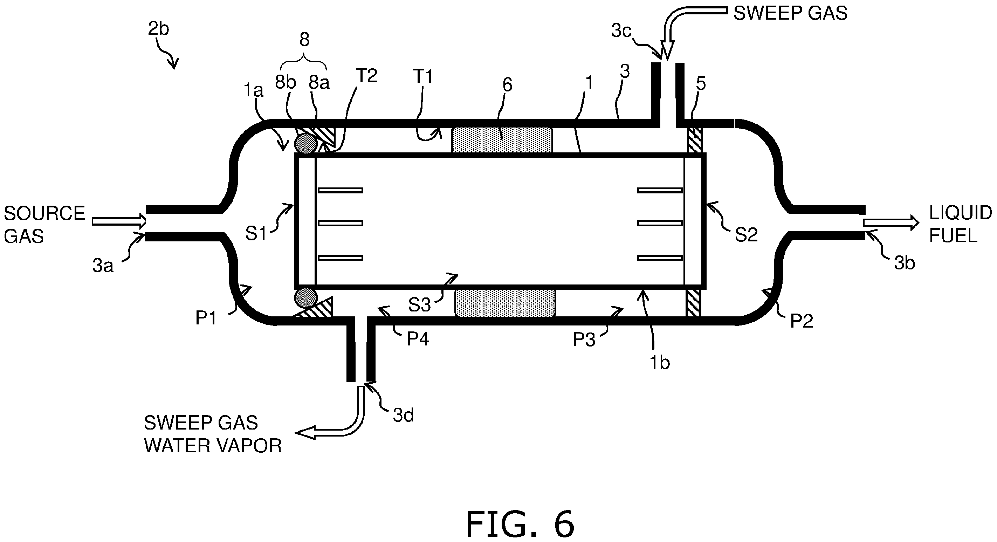

Next, the following describes a separation membrane module 2b according to a second embodiment. FIG. 6 is a transparent side view of the separation membrane module 2b. The separation membrane module 2b according to the present embodiment differs from the separation membrane module 2 according to the first embodiment in the configuration of the first sealing portion. Therefore, the following mainly describes this difference.

In the present embodiment, a case is assumed in which the coefficient of thermal expansion of the housing 3 is larger than the coefficient of thermal expansion of the reactor 1.

As shown in FIG. 6, the separation membrane module 2b includes an annular first sealing portion 8.

The first sealing portion 8 seals a gap between the housing 3 and the first end portion 1a of the reactor 1. The first sealing portion 8 holds the first end portion 1a of the reactor 1. The first sealing portion 8 includes a fixed portion 8a and an abutting portion 8b. The fixed portion 8a is an example of a "second fixed portion" according to the present invention, and the abutting portion 8b is an example of an "abutting portion" according to the present invention.

The fixed portion 8a has an annular shape. The fixed portion 8a is fixed to the inner surface T1 of the housing 3. The fixed portion 8a is disposed in such a manner as to protrude from the inner surface T1 of the housing 3. It is desirable that the fixed portion 8a is made of a material similar to the material of the housing 3, but there is no limitation to this configuration as long as the fixed portion 8a serves as a fixed portion.

The fixed portion 8a has an inclined surface T2 facing the side surface S3 of the reactor 1. The inclined surface T2 is inclined with respect to the side surface S3. Specifically, the distance between the inclined surface T2 and the side surface S3 increases as the inclined surface T2 extends toward the first end surface S1 of the reactor 1.

The abutting portion 8b has an annular shape. The abutting portion 8b is attached to the side surface S3 of the reactor 1. The abutting portion 8b abuts against the inclined surface T2 of the fixed portion 8a. The abutting portion 8b is sandwiched between the fixed portion 8a and the side surface S3 of the reactor 1.

Therefore, when the housing 3 expands in the longitudinal direction as a result of the coefficient of thermal expansion of the housing 3 being larger than the coefficient of thermal expansion of the reactor 1 when the separation membrane module 2b starts to operate, the abutting portion 8b is pressed against the inclined surface T2 of the fixed portion 8a. Since the separation membrane module can cope with the expansion and contraction of the housing 3 while maintaining the holding ability and the sealing ability of the first sealing portion 8, the reactor 1 and the first sealing portion 8 can be kept from being damaged as a result of stress being applied thereto.

### 4. Third Embodiment

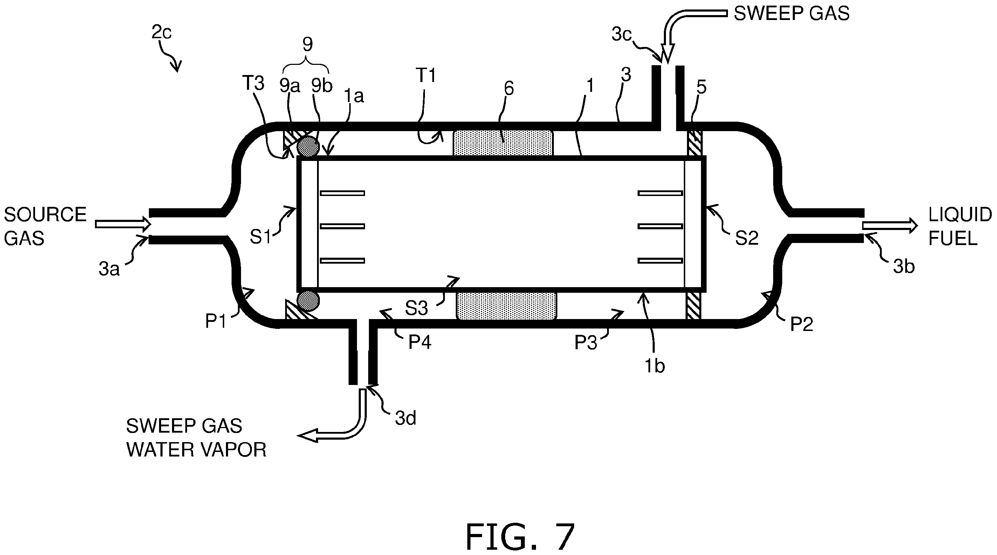

Next, the following describes a separation membrane module 2c according to a third embodiment. FIG. 7 is a transparent side view of the separation membrane module 2c. The separation membrane module 2c according to the present embodiment differs from the separation membrane module 2 according to the first embodiment in the configuration of the first sealing portion. Therefore, the following mainly describes this difference.

In the present embodiment, a case is assumed in which the coefficient of thermal expansion of the reactor 1 is larger than the coefficient of thermal expansion of the housing 3.

As shown in FIG. 7, the separation membrane module 2c includes an annular first sealing portion 9.

The first sealing portion 9 seals a gap between the housing 3 and the first end portion 1a of the reactor 1. The first sealing portion 9 holds the first end portion 1a of the reactor 1. The first sealing portion 9 includes a fixed portion 9a and an abutting portion 9b. The fixed portion 9a is an example of a "third fixed portion" according to the present invention, and the abutting portion 9b is an example of a "second abutting portion" according to the present invention.

The fixed portion 9a has an annular shape. The fixed portion 9a is fixed to the inner surface T1 of the housing 3. The fixed portion 9a is disposed in such a manner as to protrude from the inner surface T1 of the housing 3. It is desirable that the fixed portion 9a is made of a material similar to the material of the housing 3, but there is no limitation to this configuration as long as the fixed portion 9a serves as a fixed portion.

The fixed portion 9a has an inclined surface T3 facing the side surface S3 of the reactor 1. The inclined surface T3 is inclined with respect to the side surface S3. Specifically, the distance between the inclined surface T3 and the side surface S3 decreases as the inclined surface T3 extends toward the first end surface S1 of the reactor 1.

The abutting portion 9b has an annular shape. The abutting portion 9b is attached to the side surface S3 of the reactor 1. The abutting portion 9b abuts against the inclined surface T3 of the fixed portion 9a. The abutting portion 9b is sandwiched between the fixed portion 9a and the side surface S3 of the reactor 1.

Therefore, when the reactor 1 expands in the longitudinal direction as a result of the coefficient of thermal expansion of the reactor 1 being larger than the coefficient of thermal expansion of the housing 3 when the separation membrane module 2c starts to operate, the abutting portion 9b is pressed against the inclined surface T3 of the fixed portion 9a. Since the separation membrane module can cope with the expansion and contraction of the housing 3 while maintaining the holding ability and the sealing ability of the first sealing portion 9, the reactor 1 and the first sealing portion 9 can be kept from being damaged as a result of stress being applied thereto.

### Variations of Embodiments

Although the first through third embodiments of the present invention have been described, the present invention is not limited to the first through third embodiments, and various changes can be made within a scope not departing from the gist of the present invention.

### Variation 1

In the first through third embodiments described above, the separation membrane module includes the reactor, but a configuration is also possible in which the separation membrane module includes a separation filter that is used to separate a predetermined component from a fluid mixture, instead of the reactor. The configuration of the separation filter is the same as that of the reactor 1 described in the first through third embodiments, except that the separation filter includes, instead of the separation membrane 30, a separation membrane that allows a desired component to pass therethrough, and the separation filter does not include the catalyst 20. In the separation filter, the fluid mixture is supplied to the first flow paths 11. The predetermined component contained in the fluid mixture passes through the separation membrane and enters the second flow paths 12. The remaining components of the fluid mixture other than the predetermined component flows out from the first flow paths 11.

In the case where the separation membrane module includes the separation filter, reaction heat is not generated and the necessity for controlling the temperature is low, and therefore, it is possible to discharge the component that has passed through the separation membrane from the sweep gas discharge port 3d by making the pressure on the sweep gas discharge port 3d side lower than the pressure on the sweep gas supply port 3c side, without the sweep gas being used.

### Variation 2

In the first through third embodiments described above, the separation membrane 30 allows water vapor, which is one of the products generated as a result of the conversion reaction for converting the source gas into the liquid fuel, to pass therethrough, but there is not limitation to this configuration. The separation membrane 30 may also allow the liquid fuel generated as a result of the conversion reaction for converting the source gas into the liquid fuel to pass therethrough. In this case as well, it is possible to shift the reaction equilibrium of the above formula (1) to the product side.

Also, in the case where the separation membrane 30 allows the liquid fuel to pass therethrough, the reaction equilibrium can be shifted to the product side even when the liquid fuel is generated as a result of a reaction in which no water vapor is generated (e.g., 2H₂+CO ⇔ CH₃OH).

### Variation 3

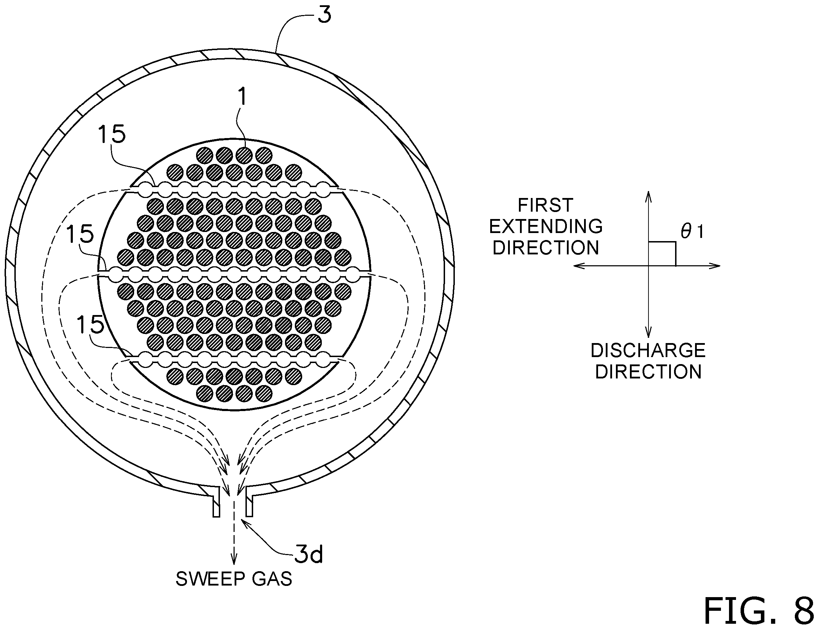

FIG. 8 is a cross-sectional view of the separation membrane module 2 shown in FIG. 5. FIG. 8 shows a cross section perpendicular to the axis of the reactor 1.

As shown in FIG. 8, a first extending direction of the outlet slits 15 extending inside the reactor 1 is preferably inclined with respect to a discharge direction of the sweep gas that is discharged from the sweep gas discharge port 3d to the outside, or the first extending direction is preferably orthogonal to the discharge direction. Specifically, an angle θ1 of the first extending direction with respect to the discharge direction is preferably 45° or more and 135° or less. With this configuration, it is possible to suppress maldistribution of gas flows from the openings on both sides of the outlet slits 15 toward the sweep gas discharge port 3b, and accordingly, it is possible to suppress maldistribution of flows of the sweep gas.

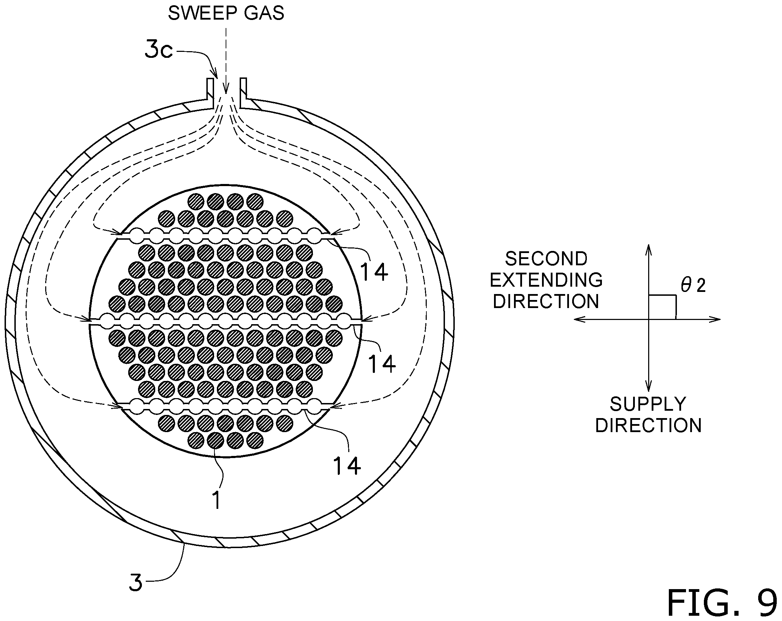

FIG. 9 is a cross-sectional view of the separation membrane module 2 shown in FIG. 5. FIG. 9 shows a cross section perpendicular to the axis of the reactor 1.

As shown in FIG. 9, a second extending direction of the inlet slits 14 extending inside the reactor 1 is preferably inclined with respect to a supply direction of the sweep gas that is supplied from the sweep gas supply port 3c, or the second extending direction is preferably orthogonal to the supply direction. Specifically, an angle θ2 of the second extending direction with respect to the supply direction is preferably 45° or more and 135° or less. With this configuration, it is possible to suppress maldistribution of gas flows from the sweep gas supply port 3c toward the openings on both sides of the inlet slits 14, and accordingly, it is possible to suppress maldistribution of flows of the sweep gas.

### Variation 4

In the first through third embodiments described above, the reactor 1 is fixed to the housing 3 (i.e., positioned in the longitudinal direction) by the second sealing portion 5 connected to each of the reactor 1 and the housing 3, but the structure for positioning the reactor 1 with use of the second sealing portion 5 is not limited to this example.

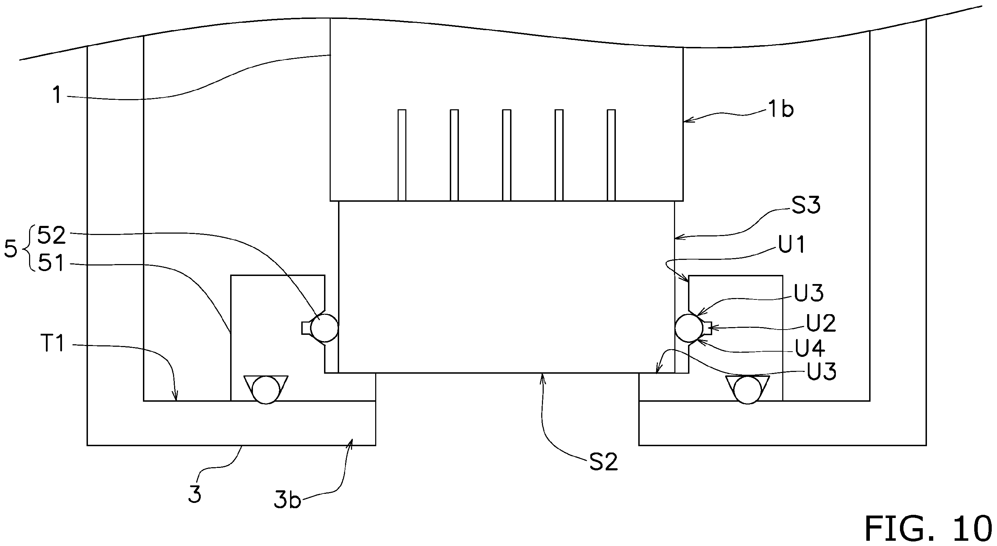

For example, as shown in FIG. 10, the second sealing portion 5 can be constituted by a flange 51 and a seal member 52.

The flange 51 is an annular member. The flange 51 is attached to the inner surface T1 of the housing 3 in such a manner as to surround the liquid fuel discharge port 3b of the housing 3. The flange 51 has a facing surface U1, an annular recess U2, and an abutting surface U3.

The facing surface U1 faces the side surface S3 of the reactor 1. The annular recess U2 is formed in the facing surface U1. The annular recess U2 includes a first inclined surface U3 and a second inclined surface U4 that abut against the seal member 52. The first inclined surface U3 is inclined with respect to the side surface S3 of the reactor 1. The distance between the first inclined surface U3 and the side surface S3 increases as the first inclined surface U3 extends toward the second end surface S2 of the reactor 1. The second inclined surface U4 is inclined with respect to the side surface S3 of the reactor 1. The distance between the second inclined surface U4 and the side surface S3 decreases as the second inclined surface U4 extends toward the second end surface S2 of the reactor 1.

The abutting surface U3 abuts against the second end surface S2 of the reactor 1. The abutting surface U3 has an annular shape. In the positioning structure shown in FIG. 10, the reactor 1 is positioned in the longitudinal direction as a result of the second end surface S2 of the reactor 1 abutting against the abutting surface U3.

The seal member 52 has an annular shape. The seal member 52 is disposed between the side surface S3 of the second end portion 1b of the reactor 1 and the first inclined surface U3 and the second inclined surface U4 of the annular recess U2 of the flange 51. The seal member 52 seals a gap between the reactor 1 and the flange 51. For example, an O ring can be used as the seal member 52.

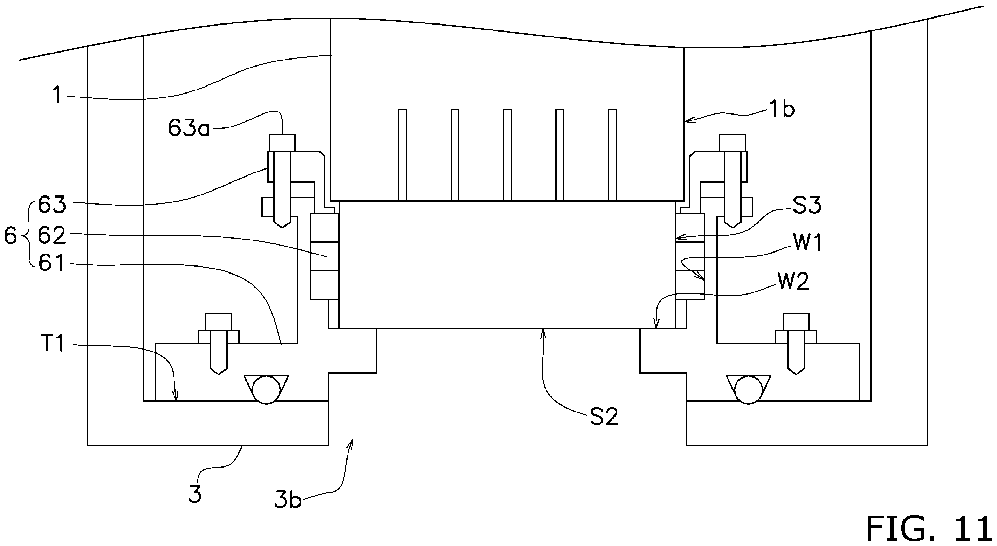

Alternatively, as shown in FIG. 11, the second sealing portion 5 can be constituted by a flange 61, a seal member 62, and a pressing member 63.

The flange 61 is an annular member. The flange 61 is attached to the inner surface T1 of the housing 3 in such a manner as to surround the liquid fuel discharge port 3b of the housing 3. The flange 61 has a facing surface W1 and an abutting surface W2.

The facing surface W1 faces the side surface S3 of the reactor 1. The abutting surface W2 abuts against the second end surface S2 of the reactor 1. The abutting surface W2 has an annular shape. In the positioning structure shown in FIG. 11, the reactor 1 is positioned in the longitudinal direction as a result of the second end surface S2 of the reactor 1 abutting against the abutting surface W2.

The seal member 62 has an annular shape. The seal member 62 is disposed between the side surface S3 of the second end portion 1b of the reactor 1 and the facing surface W1 of the flange 61. The seal member 62 seals a gap between the reactor 1 and the flange 61. The seal member 62 is a so-called gland packing. The seal member 62 can be constituted by expanded graphite, for example. The seal member 62 is compressed by the pressing member 63. The sealing ability of the seal member 62 can be adjusted by adjusting pressing force applied by the pressing member 63.

The pressing member 63 is fixed to the flange 61 by a fastening member 63a. The pressing member 63 presses the seal member 62. The pressing force applied by the pressing member 63 can be adjusted as appropriate by adjusting a fastening amount of the fastening member 63a.

### REFERENCE SIGNS LIST

1 Reactor
2, 2a to 2c Separation membrane module
3 Housing
3a Source gas supply port
3b Liquid fuel discharge port
3c Sweep gas supply port
3d Sweep gas discharge port
5 Second sealing portion
6 Flow stopper
4, 7, 8, 9 First sealing portion
4a Fixed portion (first fixed portion)
4b Elastic portion (first elastic portion)
7a Fixed portion (second fixed portion)
7b Abutting portion (first abutting portion)
7c Biasing portion
8a Fixed portion (third fixed portion)
8b Abutting portion (second abutting portion)
9a Fixed portion (third fixed portion)
9b Abutting portion (second abutting portion)
10 Porous support
11 First flow path
e1 Inlet port
e2 Outlet port
12 Second flow path
13 Cell
14 Inlet slit
d1 Inlet port
15 Outlet slit
d2 Discharge port
20 Catalyst
30 Separation Membrane
40 First seal portion
50 Second seal portion

## Claims

1. A separation membrane module comprising:
a monolith type reactor extending in a longitudinal direction and configured to be used for a conversion reaction for converting a source gas into a liquid fuel, the source gas containing hydrogen and carbon oxide;
a housing in which the reactor is housed;
an annular first sealing portion sealing a gap between the housing and a first end portion of the reactor in the longitudinal direction; and
an annular second sealing portion sealing a gap between the housing and a second end portion of the reactor in the longitudinal direction, wherein
the first sealing portion is deformable or movable in the longitudinal direction together with the reactor, and
the second sealing portion fixes the reactor to the housing.

2. The separation membrane module according to claim 1, wherein
the first sealing portion includes:
an annular first fixed portion fixed to an inner surface of the housing; and
an annular elastic portion abutting against the first fixed portion and a first end surface of the reactor.

3. The separation membrane module according to claim 1, wherein
the first sealing portion includes:
an annular second fixed portion fixed to an inner surface of the housing and having an inclined surface facing a side surface of the reactor; and
an annular abutting portion attached to the side surface and abutting against the inclined surface.

4. The separation membrane module according to claim 1, wherein
the reactor includes:
an inlet port for the source gas formed in a first end surface on the first end portion side; and
an outlet port for the liquid fuel formed in a second end surface on the second end portion side.

5. A separation membrane module comprising:
a monolith type separation filter extending in a longitudinal direction and configured to be used to separate a predetermined component from a fluid mixture;
a housing in which the separation filter is housed;
an annular first sealing portion sealing a gap between the housing and a first end portion of the separation filter in the longitudinal direction; and
an annular second sealing portion sealing a gap between the housing and a second end portion of the separation filter in the longitudinal direction, wherein
the first sealing portion is deformable or movable in the longitudinal direction together with the separation filter, and
the second sealing portion fixes the separation filter to the housing.
